# EUROPÄISCHE PATENTANMELDUNG

(11) **EP 3 381 410 A1**
(43) Veröffentlichungstag der Anmeldung: **03.10.2018**
(21) Anmeldenummer: 17164128.5
(22) Anmeldetag: 31.03.2017
(51) Int. Cl.: A61F 2/28, A61F 2/36, A61F 2/30

(54) **VERBINDUNGSHÜLSE FÜR VERANKERUNGSSCHÄFTE ZWEIER GEGENÜBERLIEGEND ANGEORDNETER PROTHESEN**

(71) Anmelder: Waldemar Link GmbH & Co. KG, 22339 Hamburg (DE)
(72) Erfinder: Link, Helmut D., 22397 Hamburg (DE); Borchers, Udo, 22850 Norderstedt (DE)
(74) Vertreter: Glawe, Delfs, Moll

(57) **Zusammenfassung**

Die Erfindung betrifft eine Verbindungshülse für Verankerungsschäfte zweier gegenüberliegend angeordneter Prothesen, vorzugsweise an einem langgestreckten Knochen wie einem Femur oder Humerus. Die Verstärkungshülse umfasst zwei Aufnahmemuffen (1, 2) für je einen Prothesenschaft und einen dazwischen angeordneten trennbaren Koppelbereich (3) zur schub- und drehfesten Verbindung. Erfindungsgemäß weisen die Aufnahmemuffen (1, 2) an ihrem dem Koppelbereich zugewandten Seite jeweils eine Gabel (31, 32) eines miteinander zusammenwirkenden Gabelpaares auf, und an einem Fuß der Gabel ist ein Passblock (4) angeordnet, dessen Seitenflächen (44) einen solchen Abstand aufweisen, der einer Innenweite der Gabel entspricht, und die Seitenflächen (44) zur flächigen Anlage an Flanken der Gabel ausgebildet sind, wobei mindestens eine Befestigungsschraube (5) quer durch die Gabel angeordnet ist. Die Gabelverbindung ist einfacher zu fertigen als die bekannte Keilverbindung und dennoch robust genug. Anders als bei der Keilverbindung ist eine exakte Passung nicht erforderlich; es genügt grundsätzlich eine Spielpassung zwischen der Gabel (31, 32) und dem Passblock (4), wobei exzessives Spiel durch die Befestigungsschraube (5) eliminiert wird.

## Beschreibung

Die Erfindung betrifft eine Verbindungshülse für Verankerungsschäfte zweier gegenüberliegend angeordneter Prothesen, vorzugsweise an einem langgestreckten Knochen wie einem Femur. Die Verstärkungshülse umfasst zwei Aufnahmemuffen für je einen Prothesenschaft und einen dazwischen angeordneten trennbaren Koppelbereich zur schub- und drehfesten Verbindung.

Insbesondere langgestreckte Knochen sind häufig mit Gelenken an beiden Enden versehen. Dies gilt insbesondere für die langgestreckten Knochen der großen Extremitäten, wie beispielsweise dem Femur oder Humerus. Aus Gründen von Krankheit oder Verschleiß kann es dazu kommen, dass an einem oder an beiden Enden künstliche Gelenke, Gelenkendoprothesen implantiert werden. In manchen Fällen geschieht dies zuerst an einem Ende des Knochens, und am anderen Ende zeitgleich, zu einem späteren Zeitpunkt oder gar nicht.

Es gibt Fälle, bei denen der langgestreckte Knochen aufgrund von Krankheit, insbesondere Knochendefekten, zu sehr geschwächt ist, um Gelenkendoprothesen an seinen Enden zu tragen. Ferner hat sich herausgestellt, dass es spätestens nach der Implantation einer zweiten Gelenkendoprothese an dem anderen Ende zu Komplikationen kommen kann. Diese können insbesondere darin bestehen, dass die in diesem Fall von beiden Enden in den langgestreckten Knochen eingesteckten Verankerungsschäfte zwar eine Verstärkung des Knochens in den jeweiligen Endbereichen bewirken (nämlich dort, wo die Schäfte eingesteckt sind), aber nicht in dem Zwischenbereich, in dem sich keiner der beiden Verankerungsschäfte befindet. Damit wird dieser Bereich, selbst wenn er biologisch an sich gesund ist, relativ geschwächt verglichen mit den durch die Verankerungsschäfte verstärkten Endbereichen. In der medizinischen Praxis hat es sich gezeigt, dass es hierbei wegen der ungleichmäßigen Kraftverteilung und der somit lokal erhöhten höheren Belastung zu Knochenfrakturen kommen kann.

Es ist ein Verstärkungsimplantat bekannt, dass an seinen Enden je eine Aufnahmemuffe für den Schaft einer Gelenkendoprothese und dazwischen ein starres Koppelstück aufweist (WO 2013/167655 A1). Das Koppelstück ist als ein Keilverbinder ausgeführt. Dieser weist zwei symmetrisch, aber gegenläufig angeordnete Keilstücke auf, deren Spitzen jeweils in taschenartige Ausnehmungen an der jeweils anderen Aufnahmemuffe eingreifen. Es wird so eine trennbare Kopplung erreicht, die im geschlossenen Zustand eine gute Kraftübertragung und hohe Betriebssicherheit aufweist. Dem steht jedoch als Nachteil gegenüber, dass für einen solchen Keilverbinder eine hohe Fertigungsgenauigkeit verlangt wird, um die gewünschte hohe Befestigungssicherheit zu erreichen. Ferner ist die Herstellung der taschenartigen Ausnehmungen ausgesprochen aufwendig, jedoch wichtig für die angestrebte Rotationssicherung. All dies setzt einer breiteren Anwendung und einer Verkleinerung, etwa zur Anwendung am Oberarmknochen, Grenzen.

Der Erfindung liegt die Aufgabe zu Grunde, ein verbessertes Verstärkungsimplantat zu schaffen, welches weniger aufwendig ist und sich besser für eine kleinere Ausführungsform eignet.

Die erfindungsgemäße Lösung liegt in den Merkmalen des unabhängigen Anspruchs. Vorteilhafte Weiterbildungen sind Gegenstand der abhängigen Ansprüche.

Bei einer Verstärkungshülse für Verankerungsschäfte zweier gegenüberliegend angeordneter Prothesen, vorzugsweise an einem langgestreckten Knochen wie einem Femur, umfassend eine erste Aufnahmemuffe für einen der Prothesenschäfte an einem Ende der Verstärkungshülse und eine zweite Aufnahmemuffe für einen zweiten der Prothesenschäfte an einem gegenüberliegenden Ende der Verstärkungshülse, und einem dazwischen angeordneten trennbaren sowie schub- und drehfest verbindbaren Koppelbereich ist erfindungsgemäß vorgesehen, dass die Aufnahmemuffen an ihrer dem Koppelbereich zugewandten Seite jeweils eine Gabel eines miteinander zusammenwirkenden Gabelpaares aufweisen, wobei jede Gabel Gabelzinken und einen Gabelgrund umfasst, und an einem Fuß der Gabel (Gabelgrund) ein Passblock angeordnet ist, dessen Seitenflächen einen solchen Abstand aufweisen, der einer Innenweite der Gabel entspricht, und die Seitenflächen zur flächigen Anlage an Flanken der Gabel ausgebildet sind, wobei mindestens eine Befestigungsschraube quer durch die Gabel angeordnet ist.

Die Erfindung beruht auf der Erkenntnis, dass eine Gabelbauweise für die Kopplung einfacher zu fertigen ist als die bekannte Keilverbindung und dennoch die Anforderungen in Bezug auf Kraftübertragung und Betriebssicherheit erfüllt. Insbesondere die kritische Sicherung gegenüber unerwünschten Verdrehungen, mithin also die Drehfestigkeit, wird durch die erfindungsgemäße Gabelkonstruktion gut erfüllt, und zwar bei spürbar geringerem Aufwand. Die Erfindung macht sich jeweils zunutze, dass - anders als bei einer Keilverbindung wie aus dem Stand der Technik bekannt - für die Gabelkonstruktion eine exakte Passung nicht erforderlich ist. Es genügt grundsätzlich eine Spielpassung zwischen der Gabel und dem Passblock, wobei exzessives Spiel durch die Befestigungsschrauben eliminiert wird. Damit erreicht die Erfindung auf elegante Weise eine Kombination von guten Kraftübertragungseigenschaften, hoher Drehfestigkeit mit geringen Anforderungen an die Fertigungstoleranzen. Dies ist im Stand der Technik ohne Beispiel.

Nachfolgend seien einige verwendete Begriffe erläutert:
Unter einer Gabel wird eine Bauweise verstanden, welche mindestens zwei langgestreckte, parallele zinkenartige Vorsprünge aufweist, die nach Art eines U angeordnet sind und zwischen sich einen Gabelgrund aufweisen. Hierbei kann der Gabelgrund auch durch ein anderes Bauteil gebildet sein, beispielsweise einen Block, der gegebenenfalls auch einstückig mit der Gabel ausgeführt sein kann.

Erfindungsgemäß wird mit geringem Aufwand eine Kraftbrücke zwischen den beiden Verankerungsschäften der Prothesen gebildet, ohne dass es auf den Zustand des Knochens im Zwischenbereich ankommt. Die Kraftbrücke ist funktional vollkommen ausreichend, so dass ggf. der Knochen im Zwischenbereich reseziert werden kann. Damit eignet sich die Erfindung insbesondere auch zur Versorgung von dünnen Knochen, wie dem Humerus.

Zweckmäßigerweise ist im Passblock eine Aufnahmebohrung für die Befestigungsschraube quer zur Erstreckungsrichtung der Gabel vorgesehen, und zwar vorzugsweise an beiden Aufnahmemuffen. Durch diese Anordnung der Aufnahmebohrung kann mittels der Befestigungsschraube zum einen eine Sicherung der erfindungsgemäßen als Gabel ausgeführten Kopplung und zum anderen eine zweckmäßige Spielreduktion erreicht werden. Dies gilt insbesondere dann, wenn die Aufnahmebohrung an beiden Aufnahmemuffen in dem jeweiligen Passblock vorgesehen ist. Vorzugsweise ist je Gabel eine eigene Befestigungsschraube vorgesehen.

Vorzugsweise sind die Lateralseiten des Passblocks komplementär zu den Gabelflanken ausgeführt. Damit fungieren die Lateralseiten als Passflächen für die Gabel, sodass eine für die Kraftübertragung günstige flächige Anlage besteht. Dies ist nicht nur generell für die Kraftübertragung von Vorteil, sondern erhöht auch den Schutz vor unerwünschten Verdrehungen, mithin also die Drehfestigkeit. Mit Vorteil ist hierbei der Abstand der Lateralseiten so gewählt, dass er dem Abstand der Gabelzinken entspricht.

Vorzugsweise sind die Lateralseiten des Passblocks zueinander geneigt ausgeführt, und zwar derart, dass sie einen geringeren Abstand in Richtung der Gabel aufweisen, also in der Richtung, in welche die Gabelzinken zeigen. Es ergibt sich somit im Querschnitt gesehen ein konischer Verlauf der Lateralseiten, wodurch zum einen das Zusammenstecken erleichtert wird und zum anderen das Erreichen von Spielfreiheit begünstigt wird. Hierbei sind mit Vorteil die Gabelflächen komplementär geneigt zu den Lateralseiten, und zwar vorzugsweise um den gleichen Winkel wie die Lateralseiten. Auf diese Weise ergibt sich eine vollflächige winkelmäßige Übereinstimmung, die optimal ist für hohe Kraftübertragung und Drehfestigkeit.

Zweckmäßigerweise ist die Aufnahmebohrung am Passblock so ausgeführt, dass sie eine Spielpassung mit der Befestigungsschraube bildet. Die Spielpassung bietet nicht den Vorteil, dass sie einfacher und mit weniger Aufwand herzustellen ist, sondern ferner bietet sie den Vorteil, dass sie sich dazu eignet, gewisse Ungenauigkeiten auszugleichen. Auf diese Weise kann durch Anziehen der Befestigungsschraube ein Ausgleich von Maßabweichungen, sei es durch Toleranzen an der Gabel oder am Passblock oder aus anderen Gründen, erreicht werden.

Gemäß einem besonderen vorteilhaften Aspekt der Erfindung, der gegebenenfalls unabhängigen Schutz verdient, ist der Abstand der Aufnahmebohrung in der Gabel zum Gabelgrund größer als der Abstand der Aufnahmebohrung im Passblock zum Gabelgrund selbst. Dem liegt die Erkenntnis zugrunde, dass auf diese Weise beim Ineinanderstecken die beiden Gabeln beim Eingriff an dem jeweils gegenüberliegend angeordneten Passblock ihre Endstellung erreichen, bevor der Gabelgrund an dem jeweils gegenüberliegenden Passblock anliegt. Damit ist sichergestellt, dass die Kraftübertragung über die Lateralseiten am Passblock erfolgt. Ein doppelter Kraftübertragungsweg, über den parallel die Kraft über den Gabelgrund direkt auf den Passblock wirken könnte, wird damit verhindert; dies ist ein großer Vorteil, da somit statisch unbestimmte Verhältnisse sicher vermieden werden können.

Zweckmäßigerweise sind die Gabel und/oder der Passblock an beiden Aufnahmemuffen symmetrisch zueinander ausgeführt. Dies vereinfacht die Herstellung und die Anwendung, da im günstigsten Fall Gleichteile auf beiden Seiten verwendet werden können. Eine Verwechslung wird damit ausgeschlossen.

Vorzugsweise ist die Weite der Gabel größer dimensioniert als die Weite des aufzunehmenden Prothesenschafts. Auf diese Weise ergeben sich günstige Kraftübertragungsverhältnisse. Die auf die Gabelzinken wirkenden Momente sind somit eher geringer als die auf den Verankerungsschaft selbst wirkenden Momente. Der Gefahr einer Überlastung des Koppelbereichs mit der Gabel wird damit auf einfache, aber effektive Weise entgegengewirkt.

Die Weite im Koppelbereich ist vorzugsweise größer als die Weite im Bereich der Aufnahmemuffe. Es wird so der Tatsache Rechnung getragen, dass im kritischen Koppelbereich eine etwas erhöhte Weite den Vorteil bietet, eine bessere Abstützung von Momenten zu ermöglichen, was günstig ist für die Knick- sowie Drehfestigkeit. Damit bleibt die Gesamtanordnung noch verhältnismäßig kompakt, so dass sie auch zur Implantation in eher dünnen langgestreckten Knochen noch geeignet ist, wie beispielsweise dem Humerus. Zweckmäßigerweise ist die Weite am Knochendurchmesser orientiert, vorzugsweise etwa gleich groß oder bis zu 10 bis 20% kleiner bzw. größer.

Zweckmäßigerweise sind die Gabel und der Passblock einstückig ausgeführt. Dies bietet den Vorteil einer einfacheren Herstellung sowie die Möglichkeit, insgesamt kleinere Abmessungen für die Gesamtanordnung zu erreichen.

Die Erfindung wird nachfolgend unter Bezugnahme auf die beigefügte Zeichnung anhand eines Ausführungsbeispiels näher erläutert. Es zeigen:
- Fig. 1: eine perspektivische Ansicht eines Ausführungsbeispiels für eine Verbindungshülse;
- Fig. 2a, b: eine Aufsicht sowie eine Seitenansicht des in Fig. 1 dargestellten Ausführungsbeispiels;
- Fig. 3: eine Darstellung der Einzelteile der Verbindungshülse gemäß dem ersten Ausführungsbeispiel;
- Fig. 4: eine Detailvergrößerung darstellend einen Koppelbereich mit Gabel und Passblock; und
- Fig. 5: eine Darstellung mit der Verbindungshülse im implantierten Zustand am Humerus.

Nachfolgend wird ein Ausführungsbeispiel für die erfindungsgemäße Verbindungshülse beschrieben. Sie umfasst jeweils eine Aufnahmemuffe 1, 2 an jedem ihrer beiden Enden sowie dazwischen einen Koppelbereich 3. Hierbei sei die mit der Bezugsziffer 1 bezeichnete Aufnahmemuffe an einer distalen Seite zugeordnet, während die mit der Bezugsziffer 2 bezeichnete Aufnahmebuchse an einer proximalen Seite zugeordnet sei.

Die Aufnahmemuffen 1, 2 sind im Wesentlichen gleich aufgebaut. Sie unterscheiden sich lediglich durch eine unterschiedliche Länge und eine unterschiedliche Weite der jeweiligen Aufnahmebohrung. Die Erläuterung erfolgt beispielhaft anhand der distal angeordneten Aufnahmemuffe 1. Sie ist im Wesentlichen von holzylindrischer Gestalt mit einem glatten Außenmantel und ist vorzugsweise aus einem biokompatiblen Material hergestellt, insbesondere einem Metall wie Titan oder Kobaltchrommolybdän (CoCrMo). Der Außendurchmesser ist abgestimmt auf die Weite des Knochens, an dem die Implantation vorgesehen ist. Meist wird der Außendurchmesser in der Regel so gewählt, dass er mit dem Durchmesser des ggf. resezierten Knochens etwa übereinstimmt. Das bedeutet, dass der Außendurchmesser nicht größer gewählt ist, als es der Breite des Knochens entspricht. In dem vorliegenden Ausführungsbeispiel ist die Verbindungshülse zur Implantation an einem Oberarmknochen (Humerus 9) vorgesehen.

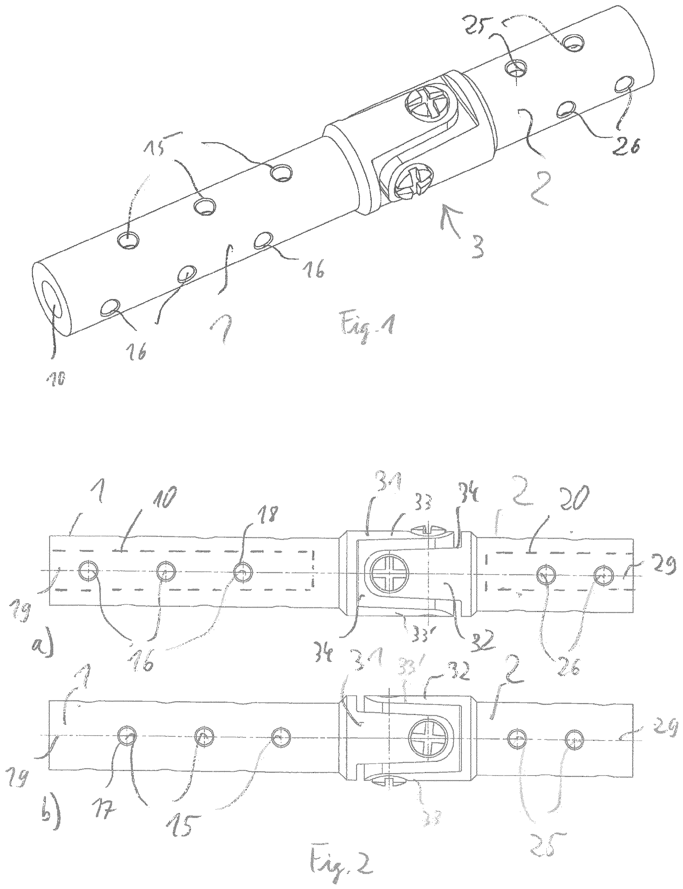

Die Aufnahmemuffe 1 weist in ihrem Innenraum einen in axialer Richtung verlaufenden Hohlraum 10 auf. Dieser ist als eine zylindrische Bohrung ausgeführt, kann aber auch anders geformt sein (konisch, Absatzbohrung etc.). Der Hohlraum 10 mündet an einer äußeren Stirnseite der Aufnahmemuffe 1. Im Mantel der Aufnahmemuffe 1 sind jeweils gegenüberliegend Paare von Lochreihen vorgesehen. An einer Vorder- bzw. Rückseite sind mehrere Löcher 15 angeordnet (siehe Figur 2b), während in axialer Richtung versetzt an den beiden Lateralseiten mehrere Löcher 16 (siehe Figur 2a) angeordnet sind. Der Versatz zwischen den Löchern 15 und 16 ist dabei so gewählt, dass das die Lochreihen etwa um die Hälfte eines Lochabstands zueinander versetzt sind. Dies ist in Figur 1 dargestellt.

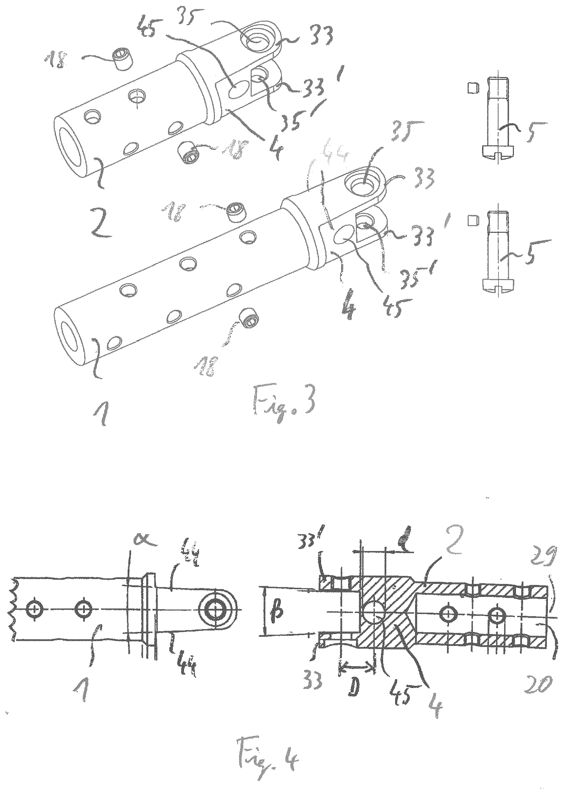

Die Aufnahmelöcher 15, 16 sind ferner so ausgerichtet, dass sie zur Mittelachse 19 des Innenraums 10 zeigen. Sie dienen zur Aufnahme von Befestigungsschrauben zur Fixierung eines in den Aufnahmeraum 10 eingeschobenen Verankerungsschafts einer Endoprothese. Die Aufnahmelöcher 15, 16 sind dazu mit jeweils einem Gewinde 17, 18 versehen zur Aufnahme von einer als Madenschraube ausgeführten Befestigungsschraube 18 (siehe Figur 3).

Die proximale Aufnahmemuffe 2 ist entsprechend ausgebildet, mit einem Hohlraum 20, Löchern 25, 26, und einer Mittelachse 29. Zur Vermeidung von Wiederholungen wird auf vorstehende Ausführungen zu der Aufnahmemuffel 1 verwiesen, die sinngemäß gelten. Im dargestellten Ausführungsbeispiel ist die Aufnahmemuffe 2 etwas kürzer als die Aufnahmemuffe 1 ausgebildet.

Der Koppelbereich 3 befindet sich an dem der jeweiligen Stirnseite gegenüberliegenden Ende der proximalen bzw. distalen Aufnahmemuffe 1, 2. An jede der beiden Aufnahmemuffe 1, 2 ist jeweils eine Gabel 31, 32 angeordnet, die jeweils zwei diametral gegenüberliegende Gabelzinken 33, 33' aufweist. Die beiden Gabelzinken 33, 33' sind jeweils spiegelbildlich ausgeführt. Sie weisen an ihrem Ende jeweils miteinander fluchtende Öffnungen 35, 35' auf, die zur Aufnahme einer Befestigungsschraube 5 dienen. Dazu weist die Öffnung 35' ein Innengewinde auf, welches zusammenwirkend mit einem Außengewinde am Bolzen der Befestigungsschraube 5 ausgeführt ist. Die Öffnung 35 ist als ein Durchgangsloch ausgebildet und weist eine Versenkung auf zur Aufnahme eines Kopfes der Befestigungsschraube 5.

Die beiden Gabelzinken 33, 33' sind U-förmig angeordnet mit einem dazwischen liegenden Gabelgrund 34. An dem Gabelgrund 34 ist j jeweils ein Passblock 4 vorgesehen. Dieser Passblock 4 kann, wie in dem dargestellten Ausführungsbeispiel, einstückig mit der jeweiligen Gabel 31, 32 ausgeführt sein. Jeder der beiden Passblöcke 4 weist eine Aufnahmebohrung 45 auf, die quer zur Längsachse 19 bzw. 29 der jeweiligen Aufnahmemuffe 1,2 ausgebildet ist. Sie dient zur Aufnahme des Schafts der Befestigungsschraube 5. Der Durchmesser der Aufnahmebohrung 43 ist so gewählt, dass eine Spielpassung mit dem Schaft der Befestigungsschraube 5 gebildet ist. Am Gewindebereich der Befestigungsschraube 5 ist jeweils ein PE-Stift 51 zur Schraubensicherung aufnehmbar.

Der Passblock 4 weist zwei gegenüberliegende Lateralseiten 44 auf, an denen Aufnahmebohrung 45 jeweils beginnt bzw. endet. Der Abstand der Lateralseiten 44 entspricht dem Abstand der beiden Gabelzinken 33,33'. Die Lateralseiten 44 sind vorzugsweise leicht schräg zueinander ausgerichtet, sodass sie an ihrer der Aufnahmebohrung 10,20 zugewandten Seite einen etwas größeren Abstand voneinander aufweisen als an ihrer den Gabelzinken 33, 33' zugewandten Seite. Zweckmäßigerweise beträgt der eingeschlossene Winkel α etwa 5°.

Dementsprechend sind die Gabelzinken 33, 33' an ihrer Innenfläche ebenfalls konisch leicht aufgeweitet. Das bedeutet, dass sie an ihrem äußeren, freien Ende einen etwas größeren Abstand aufweisen als in Richtung zum Gabelgrund 34 mit dem Passblock 4. Vorzugsweise ist hierfür ebenfalls ein eingeschlossener Winkel β gewählt, der mit dem Winkel α der Passflächen 44 übereinstimmt. Dadurch, dass diese Winkel übereinstimmen, ergibt sich eine zur Kraftübertragung vorteilhafte flächige Anlage der Innenseiten der Gabelzinken 33,33' an die Lateralseiten 44 des Passblocks 4. Ferner werden durch die Winkel gewisse Toleranzabweichungen ausgeglichen, sodass sich auch bei nicht absolut exakter Fertigung sich eine sichere kraftschlüssige Verbindung ergibt.

Die Montage erfolgt auf folgende Weise: die beiden Gabeln 31, 32 werden in eine einander gegenüberliegende Stellung gebracht, wobei die eine Gabel 31 um 90° verdreht zu der anderen Gabel 32 ist (s. Fig. 4). Die beiden Aufnahmemuffen 1, 2 mit ihren Gabeln 31, 32 werden dann so weit aufeinander zu bewegt, dass sie ineinandergreifen (s. Fig. 1). Dabei kommen die Gabelzinken 33, 33' der einen Gabel 31 zur Anlage an den Lateralflächen 44 des Passblocks 4 der gegenüberliegenden Gabel 32, und andersherum. Dadurch fluchten die Öffnungen 35, 35' jeder der beiden Gabeln 31, 32 mit der Aufnahmebohrung 45 des Passblocks 4 an der jeweils anderen Gabel 32, 31. Es wird dann jeweils eine der Befestigungsschraube 5 durch die Öffnungen 35, 35' und die Aufnahmebohrung 45 gesteckt und fest gezogen. Durch das Festziehen wird in Zusammenwirkung mit der konischen Gestaltung der Innenflächen der Gabel 31, 32 eine spielausgleichende, sichere und toleranzunempfindliche Fixierung der beiden Aufnahmemuffen 1, 2 relativ zueinander erreicht. Es ergibt sich somit eine starre und drehfeste Kraftübertragung zwischen den beiden Aufnahmemuffen 1, 2.

Im Weiteren wird Bezug genommen auf Figur 4. Dort ist das Maß für den Abstand der Lochmitte der Aufnahmebohrung 45 im Passblock 4 bezeichnen mit dem Buchstaben d. Ferner ist das entsprechende Maß für den Abstand der Lochmitte der Öffnungen 35, 35' an den Gabelzinken 33, 33' bezeichnet mit dem Maß D. Vorteilhafterweise sind die Maße so gewählt, dass das Maß D nicht gleich wie das Maß d ist, sondern etwas größer. Damit ist sichergestellt, dass die Verbindung zwischen den Gabeln 31 und 32 über die Befestigungsschrauben 5 erfolgt, und nicht die Kraft in statisch unbestimmter Weise über die Stirnseite der Passblöcke 4 geleitet ist. Auf diese Weise wird erreicht, dass die Unempfindlichkeit gegenüber Toleranzabweichungen weiter erhöht wird.

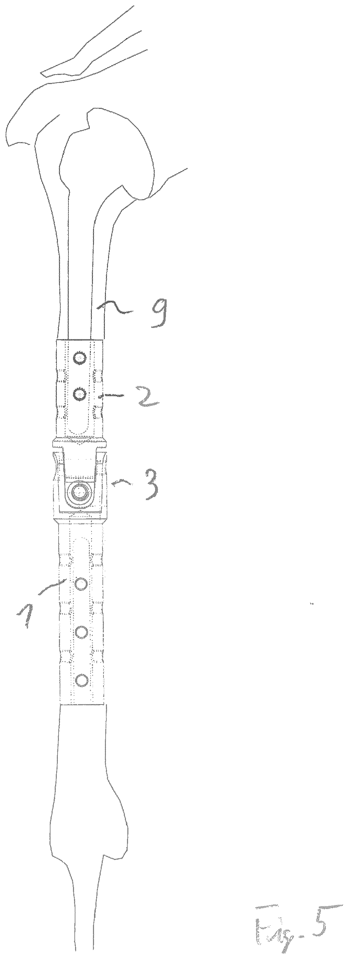

Fig. 5 zeigt die Verbindungshülse im implantierten Zustand an einem Humerus 9. Man erkennt die distal angeordnete Aufnahmemuffe 1, die proximal angeordnete Aufnahmemuffe 2 mit dem Koppelbereich 3 dazwischen. Die Weite ist im Bereich des Koppelbereichs 3 geringfügig erhöht gegenüber der Weite der Aufnahmemuffen 1, 2. Im dargestellten Ausführungsbeispiel ist die Weite etwa 10 % größer. Auf diese Weise ergibt sich eine verbesserte Befestigung der beiden Aufnahmemuffen 1, 2 relativ zueinander bei einer im praktischen Einsatz vernachlässigbaren und physiologisch gut verträglichen Ausweitung im kritischen Koppelbereich 3 zwischen den beiden Aufnahmemuffen 1, 2.

## Patentansprüche

1. Verbindungshülse für Verankerungsschäfte zweier gegenüberliegend angeordneter Prothesen, vorzugsweise an einem langgestreckten Knochen wie einem Femur, umfassend eine erste Aufnahmemuffe (1) für einen der Prothesenschäfte an einem Ende der Verstärkungshülse und eine zweite Aufnahmemuffe (2) für einen zweiten der Prothesenschäfte an einem gegenüberliegenden Ende der Verstärkungshülse, und einem dazwischen angeordneten trennbaren sowie schub- und drehfest verbindbaren Koppelbereich (3),
**dadurch gekennzeichnet, dass**
die Aufnahmemuffen (1, 2) an ihrer dem Koppelbereich (3) zugewandten Seite jeweils eine Gabel (31, 32) eines miteinander zusammenwirkenden Gabelpaares aufweisen, wobei jede Gabel (31, 32) Gabelzinken (33, 33') und einen Gabelgrund (34) umfasst, und
am Gabelgrund ein Passblock (4) angeordnet ist, dessen Seitenflächen (44) einen solchen Abstand aufweisen, der einer Innenweite der Gabel (31, 32) entspricht, und die Seitenflächen (44) zur flächigen Anlage an Flanken der Gabel (31, 32) ausgebildet sind,
wobei mindestens eine Befestigungsschraube (5) quer durch die Gabel (31, 32) angeordnet ist.

2. Verbindungshülse nach Anspruch 1, **dadurch gekennzeichnet, dass** im Passblock (4) eine Aufnahmebohrung (45) für die Befestigungsschraube (5) quer zur Erstreckungsrichtung der Gabel (31, 32) vorgesehen ist, vorzugsweise an beiden Aufnahmemuffen (1, 2).

3. Verbindungshülse nach Anspruch 1 oder 2, **dadurch gekennzeichnet, dass** die Lateralseiten des Passblocks (4) komplementär zu den Gabelflanken als Passflächen ausgeführt sind.

4. Verbindungshülse nach Anspruch 3, **dadurch gekennzeichnet, dass** der Abstand der Lateralseiten dem Abstand der Gabelzinken (33, 33') entspricht.

5. Verbindungshülse nach Anspruch 3 oder 4, **dadurch gekennzeichnet, dass** die Seitenflächen zueinander geneigt sind, mit dem geringeren Abstand in Richtung Gabel (31, 32).

6. Verbindungshülse nach Anspruch 5, **dadurch gekennzeichnet, dass** die Gabelflächen komplementär geneigt sind, vorzugsweise um den gleichen Winkel wie die Seitenflächen.

7. Verbindungshülse nach einem der vorangehenden Ansprüche, **dadurch gekennzeichnet, dass** die Aufnahmebohrung (45) am Passblock (4) eine Spielpassung mit der Befestigungsschraube (5) bildet.

8. Verbindungshülse nach einem der Ansprüche 2 bis 7, **dadurch gekennzeichnet, dass** der Abstand der Aufnahmeöffnung (35, 35') in der Gabel (31, 32) zum Gabelgrund (34) größer ist als der Abstand der Aufnahmebohrung (45) im Passblock (4) zum Gabelgrund (34).

9. Verbindungshülse nach einem der vorangehenden Ansprüche, **dadurch gekennzeichnet, dass** die Gabel (31, 32) und/oder der Passblock (4) der beiden Aufnahmemuffen symmetrisch zueinander ausgeführt sind.

10. Verbindungshülse nach einem der vorangehenden Ansprüche, **dadurch gekennzeichnet, dass** die Weite der Gabel (31, 32) größer dimensioniert ist als die Weite des aufzunehmenden Prothesenschafts.

11. Verbindungshülse nach einem der vorangehenden Ansprüche, **dadurch gekennzeichnet, dass** die Weite im Koppelbereich größer ist als die im Bereich der Aufnahmemuffen (1, 2), aber nicht mehr als ein Viertel größer, vorzugsweise maximal ein Zehntel.

12. Verbindungshülse nach einem der vorangehenden Ansprüche, **dadurch gekennzeichnet, dass** die Gabel (31, 32) und der Passblock (4) einstückig ausgeführt sind.

13. Verbindungshülse nach einem der vorangehenden Ansprüche, **dadurch gekennzeichnet, dass** für jede Gabel (31, 32) eine eigene Befestigungsschraube (5) vorgesehen ist.
